# EUROPEAN PATENT SPECIFICATION

(11) **EP 4 719 610 B1**
(45) Date of publication and mention of the grant of the patent: **18.11.2026**
(21) Application number: 24730305.0
(22) Date of filing: 29.05.2024
(51) Int. Cl.: A61Q 5/06, A61K 8/19, A61K 8/23

(54) **DYEING PRODUCT FOR KERATIN FIBERS COMPRISING AMMONIUM SALTS**
FÄRBEMITTEL FÜR KERATINFASERN MIT AMMONIUMSALZEN
PRODUIT DE COLORATION POUR FIBRES KÉRATINIQUES COMPRENANT DES SELS D'AMMONIUM

(30) Priority: 31.05.2023 EP 23176342
(43) Date of publication of application: 08.04.2026
(73) Proprietor: Kao Corporation, Tokyo 103-8210 (JP)
(72) Inventor: BREAKSPEAR, Steven, 64297 Darmstadt (DE); GHIASI, Fariba, 64297 Darmstadt (DE); NEU, Anna, 64297 Darmstadt (DE); LIPINSKI, Clarissa, 64297 Darmstadt (DE); NÖCKER, Bernd, 64297 Darmstadt (DE)
(74) Representative: Hoffmann Eitle
(86) International application number: PCT/EP2024/064767
(87) International publication number: WO 2024/246126

(56) References cited:
- EP-A1- 1 813 256
- US-A1- 2022 354 765

## Description

### Field of the invention

The present invention is directed to a dyeing product comprising at least compositions A and B wherein composition B comprises ammonium salts. A composition C for dyeing, a method, and a use are disclosed.

### Background of the invention

Direct dyes have certain safety advantages over their oxidative counterparts, but usually suffer from lower dyeing performance. Therefore, in the past many inventions focused on improving dyeing with oxidative dyes which already delivered superior dyeing intensities per se.

For example, EP3427719 discloses ammonium salts in oxidative compositions in combination with oxidative dyes. The document is silent on direct dyes and acidic compositions having low to no presence of oxidizing agents.

WO2020/127250 discloses the application of certain direct dyes in combination with alkylated ammonium salts to improve dyeing intensity. The present invention uses ammonium salts without alkylation.

Hence, despite the attempts of the prior art, the challenges with direct byes having inferior dyeing intensity and wash fastness has not been solved, yet. Thus, there is a real need to develop improved dyeing products with direct dyes.

### Summary of the invention

The first object of the present invention is a dyeing product for keratin fibers, preferably human keratin fibers, more preferably human hair, comprising at least the compositions A and B, characterized in that
- composition A comprises a) one or more hair direct dye(s), and b) one or more alkalizing agent(s), and
- composition B is an aqueous composition having a pH in the range of 1 to 6 and comprises c) one or more ammonium salt(s) of the following general formula:

   NH₄⁺ Y⁻,

   with the provision that Y⁻ is an anion except for OH⁻ and CO₃²⁻
   wherein the total concentration of oxidizing agents in composition B is 1% by weight or less, preferably 0.1% by weight or less, calculated to the total weight of composition B, more preferably composition B is free of oxidizing agents.

The second object of the present invention is a method for dyeing of keratin fibers, preferably human keratin fibers, more preferably human hair, comprising the steps of:
i) providing composition A as defined above,
ii) providing composition B as defined above,
iii) mixing the compositions of steps i) and ii) directly prior to application onto keratin fibers to yield a ready-to-use composition having a pH in the range of 7 to 12,
iv) applying the ready-to-use composition onto keratin fibers for a time period of 1 min to 60 min,
v) optionally rinsing off the keratin fibers.

The third object of the present invention is a use of a composition as defined above as composition B for enhancing color intensity and wash fastness of one or more direct dye(s) on keratin fibers, preferably human keratin fibers, more preferably human hair, with the provision that the composition B is mixed with composition A as defined above directly prior to application onto keratin fibers.

### Detailed description of the invention

Inventors of the present invention have unexpectedly and surprisingly found that a dyeing product, composition, method, and use according to the independent claims of the present invention improved the intensity of the dyeing result with direct dyes as well as their wash fastness. In addition, the dyed keratin fibers had an attractive cosmetic appearance with low to no damage.

### Dyeing product

The present invention is directed to a dyeing product for keratin fibers, preferably human keratin fibers, more preferably human hair, comprising at least the compositions A and B, characterized in that
- composition A comprises a) one or more hair direct dye(s), and b) one or more alkalizing agent(s), and
- composition B is an aqueous composition having a pH in the range of 1 to 6 and comprises c) one or more ammonium salt(s) of the following general formula:

   NH₄⁺ Y⁻,

   with the provision that Y⁻ is an anion except for OH⁻ and CO₃²⁻
   wherein the total concentration of oxidizing agents in composition B is 1% by weight or less, preferably 0.1% by weight or less, calculated to the total weight of composition B, more preferably composition B is free of oxidizing agents.

It is preferred from the viewpoint of wash fastness that the total concentration of oxidizing agents in composition A is 1% by weight or less, preferably 0.1% by weight or less, calculated to the total weight of the composition A, still more preferably composition A is free of oxidizing agents.

It is further preferred from the viewpoint of wash fastness and damage reduction that the dyeing product is a non-oxidative dyeing product.

It is preferred from the viewpoint of damage reduction that the total concentration of oxidizing agents in composition A is 1% by weight or less, preferably 0.1% by weight or less, calculated to the total weight of composition A, more preferably composition A is free of oxidizing agents. It is especially preferred that composition A is free of oxidizing bleaching compound(s) such as persalts and/or peroxy salts as well as hydrogen peroxide.

It is further preferred from the viewpoint of dyeing intensity and wash fastness that one or more ammonium salt(s) of the following general formula NH₄⁺ Y⁻, with the provision that Y⁻ is an anion except for OH⁻ and CO₃²⁻, has a molar mass of 300 g/mol or less.

It is further preferred from the viewpoint of damage reduction that the dyeing product of the present invention is a non-oxidative dyeing product. Non-oxidative within the meaning of the present invention denotes a product that is free of oxidizing agents.

In this case, composition B is free of oxidizing agents, as well as composition A.

Thus, the disclosure of the present invention is also directed to non-oxidative dyeing product for keratin fibers, preferably human keratin fibers, more preferably human hair, comprising at least the compositions A and B, characterized in that
- composition A comprises a) one or more hair direct dye(s), and b) one or more alkalizing agent(s), and
- composition B is an aqueous composition having a pH in the range of 1 to 6 and comprises c) one or more ammonium salt(s) of the following general formula NH₄⁺ Y⁻, with the provision that Y⁻ is an anion except for OH⁻ and CO₃²⁻
wherein compositions A and/or B are free of oxidizing agents.

It is further preferred from the viewpoint of damage reduction that the dyeing product of the present invention is a non-reducing dyeing product. Non-reducing within the meaning of the present invention denotes a product that is free of reducing agents.

Thus, the disclosure of the present invention is also directed to non-oxidative, non-reducing dyeing product for keratin fibers, preferably human keratin fibers, more preferably human hair, comprising at least the compositions A and B, characterized in that
- composition A comprises a) one or more hair direct dye(s), and b) one or more alkalizing agent(s), and
- composition B is an aqueous composition having a pH in the range of 1 to 6 and comprises c) one or more ammonium salt(s) of the following general formula NH₄⁺ Y⁻, with the provision that Y⁻ is an anion except for OH⁻ and CO₃²⁻
wherein composition B is free of reducing agents.

Suitable mixing ratios by weight of compositions A and B of the dyeing product of the present invention are in the range of 0.1 to 10, preferably in the range of 0.2 to 5, more preferably in the range of 0.3 to 3. The most preferred mixing ratio by weight of compositions A and B of the dyeing product of the present invention is 1.

It is preferred from the viewpoint of dyeing intensity that one or more compound(s) according to group a) of composition A is one or more non-ionic and/or anionic direct dye(s), and/or their salt(s), and/or their mixtures.

It is particularly preferred from the viewpoint of dyeing intensity that one or more compound(s) according to group a) of composition A is Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Acid Red 52, DC Violet 2, DC Red 33, DC Orange 4, DC Red 27, DC Yellow 10, HC Blue 18, HC Red 18, HC Yellow 16, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 and Basic Orange 31. The more preferred ones are Basic Red 51, Basic Yellow 87, Basic Orange 31, HC Blue 17 and Basic Blue 124, HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, HC Blue 18, HC Red 18, HC Yellow 16, Disperse Black 9, Acid Yellow 1, and/or their salt(s), and/or their mixtures.

More preferably, the compound(s) according to group a) from the viewpoint of dyeing intensity and wash fastness is/are selected from HC Blue 18, HC Red 18, HC Yellow 16, Disperse Black 9, Acid Yellow 1, and/or their salt(s), and/or their mixtures.

The most preferred compound(s) according to group a) is/are selected from HC Blue 18, HC Red 18, HC Yellow 16, and/or their salt(s), and/or their mixtures, from the viewpoint of dyeing intensity, color brilliance, and cosmetic safety.

It is preferred from the viewpoint of dyeing intensity that the total concentration of one or more compound(s) according to group a) in composition A is 0.05% by weight or more, more preferably it is 0.1% by weight or more, still more preferably it is 0.25% by weight or more, calculated to the total weight of composition A.

It is preferred from the viewpoint of cosmetic safety that the total concentration of one or more compound(s) according to group a) in composition A is 15% by weight or less, more preferably it is 12% by weight or less, still more preferably it is 10% by weight or less, calculated to the total weight of composition A.

For attaining the above-mentioned effects, it is preferred that the total concentration of one or more compound(s) according to group a) in composition A is in the range of 0.05% to 15% by weight, preferably in the range of 0.1% to 12% by weight, more preferably in the range of 0.25% to 10% by weight, calculated to the total weight of composition A.

It is further preferred from the viewpoint of dyeing intensity that one or more compound(s) according to group b) is/are selected from one or more organic alkalizing agent(s) and/or ammonium hydroxide.

Preferably one or more compound(s) according to group b) is/are one or more alkanolamine(s) and/or its/their salt(s) and/or ammonium hydroxide, more preferably one or more compound(s) according to group b) is/are selected from monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethane and/or its/their salt(s), and/or their mixtures, most preferably it is selected from monoethanolamine, 2-amino-2-methylpropanol, tris-(hydroxymethyl)-aminomethane, ammonium hydroxide, and/or their mixtures.

Ammonium hydroxide is listed as alkalizing agent according to group b) of composition A, i.e., the skilled person notices that ammonium hydroxide is not an ammonium salt within the meaning of compound(s) according to group c). Ammonium hydroxide is the aqueous solution of the gaseous compound ammonia and a salt in solid form at 25°C and atmospheric pressure cannot be harvested from the compound.

It is preferred from the viewpoint of alkalinity and dyeing intensity that the total concentration of one or more compound(s) according to group b) in composition A is 0.1% by weight or more, preferably 0.25% by weight or more, more preferably 0.5% by weight or more, calculated to the total weight of composition A.

It is preferred from the viewpoint of alkalinity, dyeing intensity, and cosmetic safety that the total concentration of one or more compound(s) according to group b) in composition A is 30% by weight or less, preferably 25% by weight or less, more preferably 20% by weight or less, calculated to the total weight of composition A.

For attaining the above-mentioned effects, it is preferred that the total concentration of one or more compound(s) according to group b) in composition A is in the range of 0.1% to 30% by weight, preferably in the range of 0.25% to 25% by weight, more preferably in the range of 0.5% to 20% by weight, calculated to the total weight of composition A.

### Product forms of composition A

In one aspect of the present invention, composition A is a powder composition and comprises water at 10% by weight or less, preferably at 1% by weight or less, more preferably at 0.1% by weight or less, calculated to the total weight of composition A, still more preferably composition A is an anhydrous powder composition.

The term 'powder' denotes a freely flowing solid composition at 25°C and atmospheric pressure consisting of individual particles or aggregates in micron and submicron range.

The term 'anhydrous' denotes an exclusion of added water. However, this does not exclude the presence of adsorbed water or crystal water in any of the powder components. In any case, the presence of a certain amount of water does not change the physical state of aggregation of the composition being a powder.

In case composition A is a powder composition, it is preferred from the viewpoint of cosmetic applicability that composition A comprises one or more pulverulent excipient. The term 'excipient' denotes a compound, which may act as filling material and dispersant for the other compounds of composition A and does not react with the dyes or the alkalizing agent, and, thus, confers the powder a certain degree of storage stability over an extended period of time.

In case composition A of the present invention is a powder composition, it may comprise an organic and/or an inorganic pulverulent excipient in which the dyes are dispersed.

Suitable organic and/or an inorganic pulverulent excipients are, for example, diatomaceous earth, kaolin, bentonite, starch especially corn, tapioca, rice, wheat and potato, nylon powder, montmorillonit, gypsum, sawdust and perlite, more preferably it is corn starch, from the viewpoint of stability.

Preferably, the total concentration of pulverulent excipients in composition A, more preferably the total concentration of organic and/or an inorganic pulverulent excipients in composition A, is 30% by weight or more, more preferably 50% by weight or more, further more preferably 60% by weight or more, still further more preferably 65% by weight or more, even further more preferably 70% by weight or more, even more preferably 75% by weight or more, calculated to the total weight of composition A, from the viewpoint of achieving good dispersability of the dyes in the powder and quick dissolution of the powder.

Preferably, the total concentration of pulverulent excipients in composition A, more preferably the total concentration of organic and/or an inorganic pulverulent excipients in composition A, is 98% by weight or less, more preferably 95% by weight or less, further more preferably 90% by weight or less, calculated to the total weight of composition A, from the viewpoint of achieving good dispersability of the dyes in the powder and formulation freedom.

For attaining the above mentioned effects, it is preferred that the total concentration of pulverulent excipients in composition A, more preferably the total concentration of organic and/or an inorganic pulverulent excipients in composition A, is in the range of 10% to 98% by weight, more preferably in the range of 15% to 95% by weight, further more preferably in the range of 20% to 90% by weight, still further more preferably in the range of 25% to 90% by weight, still further more preferably in the range of 30% to 90% by weight, even more preferably in the range of 55% to 90% by weight, calculated to the total weight of composition A.

Therefore, the disclosure of the present invention is also directed to a dyeing product for keratin fibers, preferably human keratin fibers, more preferably human hair, comprising at least the compositions A and B, characterized in that
- composition A comprises a) one or more hair direct dye(s), and b) one or more alkalizing agent(s), and
- composition B is an aqueous composition having a pH in the range of 1 to 6 and comprises c) one or more ammonium salt(s) of the following general formula NH₄⁺ Y⁻, with the provision that Y⁻ is an anion except for OH⁻ and CO₃²⁻

wherein the total concentration of oxidizing agents in composition B is 1% by weight or less, preferably 0.1% by weight or less, calculated to the total weight of composition B, more preferably composition B is free of oxidizing agents,
wherein composition A is a powder composition and comprises water at 10% by weight or less, preferably at 1% by weight or less, more preferably at 0.1% by weight or less, calculated to the total weight of composition A, still more preferably composition A is an anhydrous powder composition,
wherein composition A comprises one or more pulverulent excipient,
wherein preferably the total concentration of pulverulent excipients in composition A, more preferably the total concentration of organic and/or an inorganic pulverulent excipients in composition A, is in the range of 10% to 98% by weight, more preferably in the range of 15% to 95% by weight, further more preferably in the range of 20% to 90% by weight, still further more preferably in the range of 25% to 90% by weight, still further more preferably in the range of 30% to 90% by weight, even more preferably in the range of 55% to 90% by weight, calculated to the total weight of composition A.

In another aspect of the present invention, the composition A is a liquid composition at 25°C and atmospheric pressure comprising one or more organic solvent(s) and less than 10% by weight of water, preferably 1% by weight of water or less, more preferably 0.1% by weight of water or less, calculated to the total weight of composition A, still more preferably the composition A comprising one or more organic solvent(s) is anhydrous and liquid at 25°C and atmospheric pressure, from the viewpoint of dye stability.

The term 'liquid' denotes a physical state at 25°C and atmospheric pressure, i.e., the dyeing composition is liquid at room temperature.

For this aspect of the present invention, the composition may comprise one or more organic solvent(s).

The organic solvent(s) may be selected to dissolve the compounds a) to b). Preferred solvents are mono-, di-, and trivalent alcohols and/or their mixtures.

Preferred mono-, di-, and trivalent alcohols from the viewpoint of cosmetic safety and dissolution capacity are ethanol, n-propanol, isopropanol, propylene glycol, ethylene glycol, benzyl alcohol, phenoxyethanol, and glycerol, and/or their mixtures.

It is further preferred from the viewpoint of solution stability that the total concentration of organic solvents in composition A is 75% by weight or more, more preferably 80% by weight or more, further more preferably 85% by weight or more, calculated to the total weight of composition A.

It is further preferred from the viewpoint of dyeing intensity that the total concentration of organic solvents in composition A is 98% by weight or less, more preferably 95% by weight or less, further more preferably 92% by weight or less, calculated to the total weight of composition A.

For attaining the above-mentioned effects, it is preferred that the total concentration of organic solvents in composition A is in the range of 75% to 98% by weight, more preferably 80% to 95% by weight, further more preferably in the range of 85% to 92% by weight, calculated to the total weight of composition A.

Therefore, the disclosure of the present invention is also directed to a dyeing product for keratin fibers, preferably human keratin fibers, more preferably human hair, comprising at least the compositions A and B, characterized in that
- composition A comprises a) one or more hair direct dye(s), and b) one or more alkalizing agent(s), and
- composition B is an aqueous composition having a pH in the range of 1 to 6 and comprises c) one or more ammonium salt(s) of the following general formula NH₄⁺ Y⁻, with the provision that Y⁻ is an anion except for OH⁻ and CO₃²⁻

wherein the total concentration of oxidizing agents in composition B is 1% by weight or less, preferably 0.1% by weight or less, calculated to the total weight of composition B, more preferably composition B is free of oxidizing agents,
wherein composition A is a liquid composition at 25°C and atmospheric pressure comprising one or more organic solvent(s) and less than 10% by weight of water, preferably 1% by weight of water or less, more preferably 0.1% by weight of water or less, calculated to the total weight of composition A, still more preferably the composition A comprising one or more organic solvent(s) is anhydrous and liquid at 25°C and atmospheric pressure, preferably the total concentration of organic solvents in composition A is in the range of 75% to 98% by weight, more preferably 80% to 95% by weight, further more preferably in the range of 85% to 92% by weight, calculated to the total weight of composition A.

In another aspect of the present invention, composition A is an aqueous composition.

The term 'aqueous' denotes a composition that comprises a majority of water, i.e., the composition preferably comprises water at 50% by weight or more, further more preferably at 60% by weight or more, still more preferably at 70% by weight or more, still further more preferably at 80% by weight or more, calculated to the total weight of composition A, from the viewpoint of achieving a cosmetically acceptable composition A.

It is further preferred from the viewpoint of dyeing intensity that composition A comprises water at 98% by weight or less, more preferably at 95% by weight or less, further more preferably at 92% by weight or less, calculated to the total weight of composition A.

For achieving the above-mentioned effects, it is preferred that the total concentration of water in composition A is in the range of 50% to 98% by weight, more preferably in the range of 60% to 95% by weight, further more preferably in the range of 70% to 92% by weight, still more preferably in the range of 80% to 92% by weight, calculated to the total weight of composition A.

It is preferred from the viewpoint of dyeing performance that the pH of the aqueous composition A is 7 or more, more preferably the pH is 7.5 or more, further more preferably the pH is 8 or more.

It is preferred from the viewpoint of hair damage and dyeing performance that the pH of the aqueous composition A is 12 or less, more preferably the pH is 11.5 or less, still more preferably the pH is 11 or less.

For attaining the above mentioned effects, it is preferred that the pH of the aqueous composition A is in the range of 7 to 12, preferably in the range of 7.5 to 11.5, more preferably in the range of 8 to 11.

### Composition B

The dyeing product of the present invention comprises a composition B as an aqueous composition having a pH in the range of 1 to 6 and comprises c) one or more ammonium salt(s) of the following general formula NH₄⁺ Y⁻, with the provision that Y⁻ is an anion except for OH⁻ and CO₃²⁻,
wherein the total concentration of oxidizing agents in composition B is 1% by weight or less, preferably 0.1% by weight or less, calculated to the total weight of composition B, more preferably composition B is free of oxidizing agents.

It is preferred from the viewpoint of cosmetic acceptance that the pH of composition B is 1.5 or more, more preferably 2.0 or more, still more preferably 2.5 or more.

It is preferred from the viewpoint of stability that the pH of composition B is 5.5 or less, more preferably 5.0 or less, still more preferably 4.5 or less.

For attaining the above-mentioned effects, it is preferred that the pH of composition B is in the range of 1.5 to 5.5, more preferably in the range of 2.0 to 5.0, still more preferably in the range of 2.5 to 4.5.

Composition B comprises one or more ammonium salt(s) of the following general formula NH₄⁺ Y⁻, with the provision that Y⁻ is an anion except for OH⁻ and CO₃²⁻, as compound(s) according to group c).

Suitable compound(s) according to group c) is/are ammonium sulfate, ammonium chloride, ammonium acetate, ammonium benzoate, ammonium carbamate, ammonium chloride, ammonium ferric pentetate, ammonium fluoride, ammonium iodide, ammonium bromide, ammonium lactate, ammonium nitrate, ammonium phosphate, diammonium phosphate, triammonium phosphate, ammonium propionate, ammonium sulfite, ammonium thiocyanate, ammonium xylenesulfonate, ammonium citrate, diammonium citrate, triammonium citrate, and/or their mixtures.

It is preferred from the viewpoint of cosmetic acceptance and stability that one or more compound(s) according to group c) is ammonium sulfate, ammonium chloride, ammonium phosphate, diammonium phosphate, triammonium phosphate, and/or their mixtures, more preferably one or more compound(s) according to group c) is ammonium sulfate.

It is preferred from the viewpoint of dyeing intensity that the total concentration of one or more compound(s) according to group c) in composition B is 0.1% by weight or more, more preferably 0.5% by weight or more, still more preferably 0.75% by weight or more, still more preferably 1% by weight or more, calculated to the total weight of composition B.

It is preferred from the viewpoint of smell and cosmetic acceptance that the total concentration of one or more compound(s) according to group c) in composition B is 15% by weight or less, more preferably 10% by weight or less, still more preferably 8% by weight or less, still more preferably 6% by weight or less, calculated to the total weight of composition B.

For attaining the above-mentioned effects, it is preferred that the total concentration of one or more compound(s) according to group c) in composition B is in the range of 0.1% to 15% by weight, preferably in the range of 0.5% to 10% by weight, more preferably in the range of 0.75% to 8% by weight, still more preferably in the range of 1% to 6% by weight, calculated to the total weight of composition B.

It is preferred from the viewpoint of user friendliness and dyeing intensity that compositions A and/or B comprise one or more lipophilic compound(s) at 25°C and atmospheric pressure as compound(s) according to group d).

Preferably one or more compound(s) according to group d) is/are selected from C₁₂ to C₂₂ fatty alcohols, esters of C₃ to C₂₂ alcohols with C₁₂ to C₂₂ fatty acids, C₈ to C₂₂ fatty acids, vegetable oils, and/or silicones, and/or hydrocarbon-based products, and/or their mixtures.

Suitable C₁₂ to C₂₂ fatty alcohols are are myristyl alcohol, cetyl alcohol, stearyl alcohol, behenyl alcohol, and cetearyl alcohol, and/or their mixtures.

Suitable esters of C₃ to C₂₂ alcohols with C₁₂ to C₂₂ fatty acids are isopropyl myristate, isopropyl palmitate, and myristyl myristate, and/or their mixtures.

Suitable C₈ to C₂₂ fatty acids are oleic acid, linoleic acid, and palmitic acid, and/or their mixtures.

Suitable vegetable oils are olive oil, almond oil, sunflower oil, and argan oil, and/or their mixtures.

Suitable silicones are non-aminated and/or aminated silicones. The latter are commonly known as amodimethicones.

It is preferred from the viewpoint of composition stability and dyeing intensity that the total concentration of one or more compound(s) according to group d) in compositions A and/or B is 1% by weight or more, more preferably 2% by weight or more, further more preferably 3% by weight or more, calculated to the total weight of each of the compositions A and/or B.

It is preferred from the viewpoint of forming a stable composition that the total concentration of one or more compound(s) according to group d) in compositions A and/or B is 20% by weight or less, more preferably 15% by weight or less, further more preferably 12% by weight or less, calculated to the total weight of each of the compositions A and/or B.

For attaining the above-mentioned effects, it is preferred that the total concentration of one or more compound(s) according to group d) in compositions A and/or B is in the range of 1% to 20% by weight, preferably in the range of 2% to 15% by weight, more preferably in the range of 3% to 12% by weight, calculated to the total weight of each of the compositions A and/or B.

It is preferred from the viewpoint of user friendliness and dyeing intensity that composition B is a water-in-oil emulsion comprising one or more compound(s) according to group d).

It is preferred from the viewpoint of product stability that compositions A and B are kept separate until directly prior to application onto keratin fibers.

When compositions A and B are mixed prior to application onto keratin fibers, it is preferred from the viewpoint of dyeing intensity that the pH of the ready-to-use composition is 7 or more, preferably 7.5 or more, more preferably 8 or more.

When compositions A and B are mixed prior to application onto keratin fibers, it is preferred from the viewpoint of dyeing intensity and cosmetic safety that the pH of the ready-to-use composition is 12 or less, preferably 11.5 or less, more preferably 11 or less.

For attaining the above-mentioned effects, it is preferred that the pH of the ready-to-use composition yielded by mixing compositions A and B is in the range of 7 to 12, preferably in the range of 7.5 to 11.5, more preferably in the range of 8 to 11.

It is further preferred from the viewpoint of damage reduction that the ready-to-use composition yielded by mixing compositions A and B comprises 1% by weight or less of oxidizing agents, preferably 0.1% or less by weight of oxidizing agents, calculated to the total weight of the ready-to-use composition prepared by mixing compositions A and B, more preferably the ready-to-use composition is free of oxidizing agents.

### Optional ingredients - surfactants

The compositions A and/or B of the present invention may further comprise one or more surfactant(s), preferably selected from non-ionic surfactants, anionic surfactants, cationic surfactants, and/or amphoteric/zwitterionic surfactants, and/or their mixtures, more preferably selected from anionic surfactants, from the viewpoint of stabilizing the composition and improving miscibility with each other.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts.

Suitable examples are alkyl sulfate or preferably ethoxylated alkyl ether sulfate surfactants or mixtures thereof, and/or salts thereof, having an alkyl chain length of C₁₀ to C₂₂ and an ethoxylation degree from 1 to 50.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants, and/or their salts. Suitable examples are cetrimonium chloride and behentrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable concentration ranges for surfactants are in the range of 0.1% to 10% by weight, calculated to the total weight of each of the compositions A and/or B, from the viewpoint of enhancing wettability of keratin fibers, physical stability, and mixability with other compositions.

### Thickening polymers

From the viewpoint of cosmetic safety, it is further preferred that the compositions A and/or B of the present invention may comprise one or more thickening polymer(s).

The compositions A and/or B of the present invention may comprise(s) one or more thickening polymer(s) selected from non-ionic thickening polymers and/or anionic thickening polymers, and/or their mixtures.

Preferably, the thickening polymers are selected from polymers resulting in an aqueous solution and/or aqueous dispersion at pH between 7 and 12 having a viscosity of at least 1,000 mPa•s measured at a polymer concentration of 1% by weight in water at 25°C under atmospheric conditions, calculated to the total weight of the composition, determined by a Brookfield viscometer, such as at 10 rpm for 1 min with spindle #4 at 25°C.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethyl-methylcellulose, and alkylated hydroxyl celluloses such as (C₂-C₈)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch-based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

Suitable synthetic anionic polymers are associative thickening polymers, such as acrylates/steareth-30 methacrylate copolymer.

The preferred thickening polymer for the composition of the present invention are natural anionic polymers, more preferably xanthan gum and/or dehydroxanthan gum, from the viewpoint of their biodegradability and low environmental impact.

Preferably, the total concentration of thickening polymers in the compositions A and/or B of the present invention is/are 0.1% by weight or more, more preferably 0.25% by weight or more, more preferably 0.5% by weight or more, calculated to the total weight of each of the compositions A and/or B, from the viewpoint of providing sufficient viscosity to compositions A and/or B.

Preferably, the total concentration of thickening polymers in the compositions A and/or B of the present invention is 15% by weight or less, more preferably 12% by weight or more, further more preferably 10% by weight or less, calculated to the total weight of each of the compositions A and/or B, from the viewpoint of providing sufficient viscosity to the compositions A and/or B and cost of goods.

For attaining the above-mentioned effects, it is preferred that the total concentration of thickening polymers in the compositions A and/or B of the present invention is in the range of 0.1% to 15% by weight, preferably in the range of 0.25% to 12% by weight, more preferably in the range of 0.5% to 10% by weight, calculated to the total weight of each of the compositions A and/or B.

It is preferred from the viewpoint of cosmetic safety that the compositions A and/or B of the present invention has/have a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions. A suitable viscometer is a Brookfield viscometer with spindle #4.

### Method for dyeing

The present invention is also directed to a method for dyeing of keratin fibers, preferably human keratin fibers, more preferably human hair, comprising the steps of:
i) providing composition A as defined above,
ii) providing composition B as defined above,
iii) mixing the compositions of steps i) and ii) directly prior to application onto keratin fibers to yield a ready-to-use composition having a pH in the range of 7 to 12,
iv) applying the ready-to-use composition onto keratin fibers for a time period of 1 min to 60 min,
v) optionally rinsing off the keratin fibers.

It is preferred from the viewpoint of accelerated dyeing that the pH of the ready-to-use composition is 7 to 12, preferably in the range of 7.5 to 11.5, more preferably in the range of 8 to 11.

The ready-to-use composition is then applied to keratin fibers and left for a time period of 1 min to 60 min as defined in step iv). Preferred time ranges for step iv) are 5 min to 45 min, more preferred ranges are 10 min to 35 min, from the viewpoint of sufficiently dyeing.

Optionally, heat may be applied while leaving the ready-to-use composition onto keratin fibers. Suitable temperature ranges are 30°C to 50°C.

### Use of composition

The present invention is also directed to a use of a composition as defined as composition B as defined above for enhancing color intensity and wash fastness of one or more direct dye(s) on keratin fibers, preferably human keratin fibers, more preferably human hair, with the provision that the composition B is mixed with composition A as defined above directly prior to application onto keratin fibers.

The disclosure of the present invention also relates to <1> a dyeing product for keratin fibers, preferably human keratin fibers, more preferably human hair, comprising at least the compositions A and B, characterized in that
- composition A comprises a) one or more hair direct dye(s), and b) one or more alkalizing agent(s), and
- composition B is an aqueous composition having a pH in the range of 1 to 6 and comprises c) one or more ammonium salt(s) of the following general formula:

   NH₄⁺ Y⁻,

   with the provision that Y⁻ is an anion except for OH⁻ and CO₃²⁻
   wherein the total concentration of oxidizing agents in composition B is 1% by weight or less, preferably 0.1% by weight or less, calculated to the total weight of composition B, more preferably composition B is free of oxidizing agents.

<2> The product according to clause <1> characterized in that the total concentration of oxidizing agents in composition A is 1% by weight or less, preferably 0.1% by weight or less, calculated to the total weight of the composition A, still more preferably composition A is free of oxidizing agents.
<3> The product according to any of the clauses <1> to <2> characterized in that the dyeing product is a non-oxidative dyeing product.
<4> The product according to any of the clauses <1> to <3> characterized in that one or more compound(s) according to group a) is one or more non-ionic and/or anionic direct dye(s), and/or their salt(s), and/or their mixtures, preferably one or more compound(s) according to group a) is Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Acid Red 52, DC Violet 2, DC Red 33, DC Orange 4, DC Red 27, DC Yellow 10, HC Blue 18, HC Red 18, HC Yellow 16, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 and Basic Orange 31, preferably Basic Red 51, Basic Yellow 87, Basic Orange 31, HC Blue 17 and Basic Blue 124, HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, HC Blue 18, HC Red 18, HC Yellow 16, Disperse Black 9, Acid Yellow 1, and/or their salt(s), and/or their mixtures.
<5> The product according to any of the clauses <1> to <4> characterized in that the one or more compound(s) according to group a) is/are selected from HC Blue 18, HC Red 18, HC Yellow 16, Disperse Black 9, Acid Yellow 1, and/or their salt(s), and/or their mixtures.
<6> The product according to any of the clauses <1> to <5> characterized in that the one or more compound(s) according to group a) is/are selected from HC Blue 18, HC Red 18, HC Yellow 16, and/or their salt(s), and/or their mixtures.
<7> The product according to any of the clauses <1> to <6> characterized in that the total concentration of one or more compound(s) according to group a) in composition A is in the range of 0.05% to 15% by weight, preferably in the range of 0.1% to 12% by weight, more preferably in the range of 0.25% to 10% by weight, calculated to the total weight of composition A.
<8> The product according to any of the clauses <1> to <7> characterized in that one or more compound(s) according to group b) is/are selected from one or more organic alkalizing agent(s), and/or their salt(s), and/or ammonium hydroxide.
<9> The product according to any of the clauses <1> to <8> characterized in that the one or more compound(s) according to group b) is selected from monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethane, ammonium hydroxide, and/or their mixtures.
<10> The product according to any of the clauses <1> to <9> characterized in that the one or more compound(s) according to group b) is selected from monoethanolamine, 2-amino-2-methylpropanol, tris-(hydroxymethyl)-aminomethane, ammonium hydroxide, and/or their mixtures.
<11> The product according to any of the clauses <1> to <10> characterized in that the total concentration of one or more compound(s) according to group b) in composition A is in the range of 0.1% to 30% by weight, preferably in the range of 0.25% to 25% by weight, more preferably in the range of 0.5% to 20% by weight, calculated to the total weight of composition A.
<12> The product according to any of the clauses <1> to <11> characterized in that composition A is a powder composition and comprises water at 10% by weight or less, preferably at 1% by weight or less, more preferably at 0.1% by weight or less, calculated to the total weight of composition A, still more preferably composition A is an anhydrous powder composition.
<13> The product according to clause <12> characterized in that composition A comprises one or more organic and/or inorganic pulverulent excipient(s).
<14> The product according to any of the clauses <12> and/or <13> characterized in that one or more organic and/or an inorganic pulverulent excipients are diatomaceous earth, kaolin, bentonite, one or more starch(es) such as corn, tapioca, rice, wheat and potato, nylon powder, montmorillonit, gypsum, sawdust and perlite, more preferably it is corn starch.
<15> The product according to any of the clauses <12> to <14> characterized in that the total concentration of pulverulent excipients in composition A, more preferably the total concentration of organic and/or an inorganic pulverulent excipients in composition A, is in the range of 10% to 98% by weight, more preferably in the range of 15% to 95% by weight, further more preferably in the range of 20% to 90% by weight, still further more preferably in the range of 25% to 90% by weight, still further more preferably in the range of 30% to 90% by weight, even more preferably in the range of 55% to 90% by weight, calculated to the total weight of composition A.
<16> The product according to any of the clauses <1> to <11> characterized in that composition A is a liquid composition at 25°C and atmospheric pressure comprising one or more organic solvent(s) and less than 10% by weight of water, preferably 1% by weight of water or less, more preferably 0.1% by weight of water or less, calculated to the total weight of composition A, still more preferably the composition A comprising one or more organic solvent(s) is anhydrous and liquid at 25°C and atmospheric pressure.
<17> The product according to clause <16> characterized in that the one or more organic solvents of composition A are mono-, di-, and trivalent alcohols, and/or their mixtures.
<18> The product according to any of the clauses <16> to <17> characterized in that the one or more organic solvent(s) are ethanol, n-propanol, isopropanol, propylene glycol, ethylene glycol, benzyl alcohol, phenoxyethanol, and glycerol, and/or their mixtures.
<19> The product according to any of the clauses <16> to <18> characterized in that that the total concentration of organic solvents in composition A is in the range of 75% to 98% by weight, more preferably 80% to 95% by weight, further more preferably in the range of 85% to 92% by weight, calculated to the total weight of composition A.
<20> The product according to any of the clauses <1> to <11> characterized in that composition A is an aqueous composition having a pH in the range of 7 to 12, preferably in the range of 7.5 to 11.5, more preferably in the range of 8 to 11.
<21> The product according to any of the clauses <1> to <11> and/or <20> characterized in that composition A comprises water at 50% by weight or more, more preferably at 60% by weight or more, still more preferably at 70% by weight or more, still furthermore preferably at 80% by weight or more, calculated to the total weight of composition A.
<22> The product according to any of the clauses <1> to <21> characterized in that one or more compound(s) according to group c) is ammonium sulfate, ammonium chloride, ammonium acetate, ammonium benzoate, ammonium carbamate, ammonium chloride, ammonium ferric pentetate, ammonium fluoride, ammonium iodide, ammonium bromide, ammonium lactate, ammonium nitrate, ammonium phosphate, diammonium phosphate, triammonium phosphate, ammonium propionate, ammonium sulfite, ammonium thiocyanate, ammonium xylenesulfonate, ammonium citrate, diammonium citrate, triammonium citrate, and/or their mixtures.
<23> The product according to any of the clauses <1> to <22> characterized in that the one or more compound(s) according to group c) is ammonium sulfate, ammonium chloride, ammonium phosphate, diammonium phosphate, triammonium phosphate, and/or their mixtures.
<24> The product according to any of the clauses <1> to <23> characterized in that the one or more compound(s) according to group c) is ammonium sulfate.
<25> The product according to any of the clauses <1> to <24> characterized in that the total concentration of one or more compound(s) according to group c) in composition B is in the range of 0.1% to 15% by weight, preferably in the range of 0.5% to 10% by weight, more preferably in the range of 0.75% to 8% by weight, still more preferably in the range of 1% to 6% by weight, calculated to the total weight of composition B.
<26> The product according to any of the clauses <1> to <25> characterized in that composition B comprises one or more lipophilic compound(s) at 25°C and atmospheric pressure as compound(s) according to group d), preferably one or more compound(s) according to group d) is/are selected from C₁₂ to C₂₂ fatty alcohols, esters of C₃ to C₂₂ alcohols with C₁₂ to C₂₂ fatty acids, C₈ to C₂₂ fatty acids, vegetable oils, and/or silicones, and/or hydrocarbon-based products, and/or their mixtures, more preferably composition B is a water-in-oil emulsion comprising one or more compound(s) according to group d).
<27> The product according to any of the clauses <1> to <25> characterized in that the pH of the ready-to-use composition yielded by mixing compositions A and B is in the range of 7 to 12, preferably in the range of 7.5 to 11.5, more preferably in the range of 8 to 11.

The present disclosure is also directed to <28> a method for dyeing of keratin fibers, preferably human keratin fibers, more preferably human hair, comprising the steps of:
i) providing composition A as defined in any of the clauses <1> to <27>,
ii) providing composition B as defined in any of the clauses <1> to <27>,
iii) mixing the compositions of steps i) and ii) directly prior to application onto keratin fibers to yield a ready-to-use composition having a pH in the range of 7 to 12,
iv) applying the ready-to-use composition onto keratin fibers for a time period of 1 min to 60 min,
v) optionally rinsing off the keratin fibers.

The present disclosure is also directed to <29> the use of a composition B as defined in any of the clauses <1> to <27> for enhancing color intensity and wash fastness of one or more direct dye(s) on keratin fibers, preferably human keratin fibers, more preferably human hair, with the provision that compositions B is mixed with composition A as defined in any of the clauses <1> to <27> directly prior to application onto keratin fibers.

The following examples are to illustrate the present invention and not to limit it.

### EXAMPLES

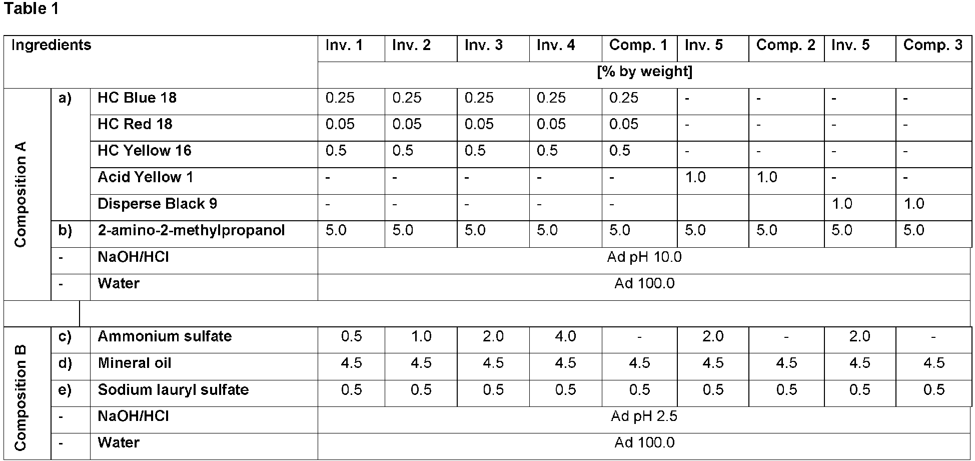

**Table 2**

| **Results** | **Inv. 1** | **Inv. 2** | **Inv. 3** | **Inv. 4** | **Comp. 1** | **Inv. 5** | **Comp. 2** | **Inv. 6** | **Comp. 3** |
|---|---|---|---|---|---|---|---|---|---|
| **Color upon dyeing (ΔE)** | 50.61 | 54.79 | 53.60 | 53.93 | 49.96 | 55.12 | 51.33 | 80.46 | 76.00 |
| **Color upon 20x washing (ΔE)** | 37.95 | 41.00 | 39.39 | 39.43 | 34.78 | 26.63 | 20.44 | 65.92 | 53.79 |
| **Loss on color (ΔΔE)** | 12.66 | 13.79 | 14.21 | 14.50 | 15.18 | 28.49 | 30.89 | 14.54 | 22.21 |

The inventive compositions yielded better dyeing intensities and wash fastness with respect to the comparative counterparts where composition B did not have an ammonium salt.

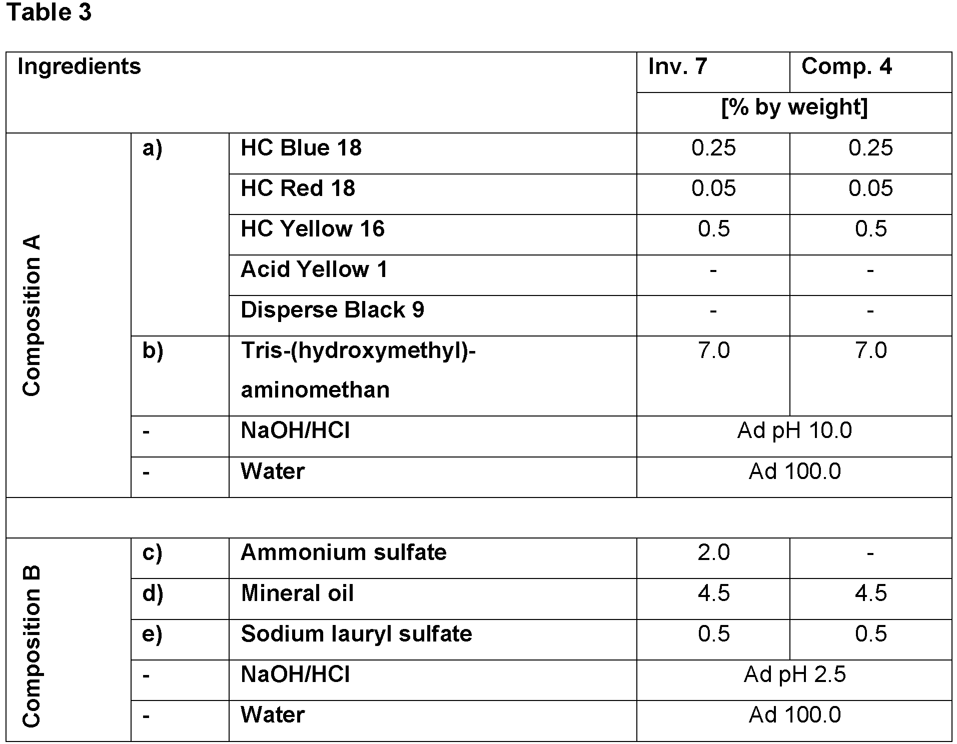

**Table 4**

| **Results** | **Inv. 6** | **Comp. 4** |
|---|---|---|
| **Color upon dyeing (ΔE)** | 68.14 | 66.04 |
| **Color upon 20x washing (ΔE)** | 62.07 | 57.74 |
| **Loss on color (ΔΔE)** | 6.07 | 8.30 |

### Methods

### Hair dyeing

Goat hairstreaks (21 cm, 2 g per bundle) were permed with a commercial perming product available under the trade name Goldwell Topform Type 1 for 20 min, rinsed, and then treated with an oxidative composition for 10 min for perm fixing. The hair streaks were shampooed and dried.

The dyeing product from above was prepared by mixing compositions A and B in a weight ratio of 1:1 to prepare ready-to-use compositions having a pH in the range of 10.0 to 10.5.

2 g of the ready-to-use compositions were applied onto the permed goat hairstreaks for 30 min at 30°C. The streaks were then rinsed off and dried. The colorimetric data were obtained with a color-difference meter (Datacolor Mercury Check II Plus) in the CIE colorimetric system (L*,a*,b*). Color differences (ΔE) were calculated by the following formula. $Δ E = \sqrt{{\left({L}_{1}^{∗}-{L}_{0}^{∗}\right)}^{2} + {\left({a}_{1}^{∗}-{a}_{0}^{∗}\right)}^{2} + {\left({b}_{1}^{∗}-{b}_{0}^{∗}\right)}^{2}}$

ΔΔE is the color difference of freshly colored hair and hair being washed for 20 times as explained below.

### Wash fastness

To the dyed hair streaks 1 g of commercial shampoo was applied available under the trade name Goldwell Dualsenses Color Fade Stop Shampoo and it was foamed up with lukewarm water for 30 s. The hair streaks were then rinsed off. These steps were repeated for 20 times. After treatment, the hairstreaks were blow-dried and the remaining color was measured (L*,a*,b*). ΔΔE was calculated as the color difference between 20 times washed and freshly colored hair.

## Claims

1. A dyeing product for keratin fibers, preferably human keratin fibers, more preferably human hair, comprising at least the compositions A and B, **characterized in that**
- composition A comprises a) one or more hair direct dye(s), and b) one or more alkalizing agent(s), and
- composition B is an aqueous composition having a pH in the range of 1 to 6 and comprises c) one or more ammonium salt(s) of the following general formula:
NH₄⁺ Y⁻,
with the provision that Y⁻ is an anion except for OH⁻ and CO₃²⁻
wherein the total concentration of oxidizing agents in composition B is 1% by weight or less, preferably 0.1% by weight or less, calculated to the total weight of composition B, more preferably composition B is free of oxidizing agents.

2. The product according to claim 1 **characterized in that** the total concentration of oxidizing agents in composition A is 1% by weight or less, preferably 0.1% by weight or less, calculated to the total weight of the compositions A, still more preferably composition A is free of oxidizing agents, still more preferably the dyeing product is a non-oxidative dyeing product.

3. The product according to any of the claims 1 and/or 2 **characterized in that** one or more compound(s) according to group a) is one or more non-ionic and/or anionic direct dye(s), and/or their salt(s), and/or their mixtures, preferably one or more compound(s) according to group a) is Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Acid Red 52, DC Violet 2, DC Red 33, DC Orange 4, DC Red 27, DC Yellow 10, HC Blue 18, HC Red 18, HC Yellow 16, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 and Basic Orange 31, preferably one or more compound(s) according to group a) is one or more Basic Red 51, Basic Yellow 87, Basic Orange 31, HC Blue 17 and Basic Blue 124, HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, HC Blue 18, HC Red 18, HC Yellow 16, Disperse Black 9, Acid Yellow 1, and/or their salt(s), and/or their mixtures, more preferably one or more compound(s) according to group a) is/are selected from HC Blue 18, HC Red 18, HC Yellow 16, Disperse Black 9, Acid Yellow 1, and/or their salt(s), and/or their mixtures, still more preferably one or more compound(s) according to group a) is/are selected from HC Blue 18, HC Red 18, HC Yellow 16, and/or their salt(s), and/or their mixtures.

4. The product according to any of the preceding claims **characterized in that** the total concentration of one or more compound(s) according to group a) in composition A is in the range of 0.05% to 15% by weight, preferably in the range of 0.1% to 12% by weight, more preferably in the range of 0.25% to 10% by weight, calculated to the total weight of composition A.

5. The product according to any of the preceding claims **characterized in that** one or more compound(s) according to group b) is/are selected from one or more organic alkalizing agent(s), and/or their salt(s), and/or ammonium hydroxide, preferably one or more compound(s) according to group b) is one or more alkanolamine(s) and/or its/their salt(s) and/or ammonium hydroxide, more preferably one or more compound(s) according to group b) is selected from monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethane, ammonium hydroxide, and/or their mixtures, most preferably one or more compound(s) according to group b) is selected from monoethanolamine, 2-amino-2-methylpropanol, tris-(hydroxymethyl)-aminomethane, ammonium hydroxide, and/or their mixtures.

6. The product according to any of the preceding claims **characterized in that** the total concentration of one or more compound(s) according to group b) in composition A is in the range of 0.1% to 30% by weight, preferably in the range of 0.25% to 25% by weight, more preferably in the range of 0.5% to 20% by weight, calculated to the total weight of composition A.

7. The product according to any of the preceding claims **characterized in that** composition A is a powder composition and comprises water at 10% by weight or less, preferably at 1% by weight or less, more preferably at 0.1% by weight or less, calculated to the total weight of composition A, still more preferably composition A is an anhydrous powder composition.

8. The product according to any of the claims 1 to 6 **characterized in that** composition A is a liquid composition at 25°C and atmospheric pressure comprising one or more organic solvent(s) and less than 10% by weight of water, preferably 1% by weight of water or less, more preferably 0.1% by weight of water or less, calculated to the total weight of composition A, still more preferably the composition A comprising one or more organic solvent(s) is anhydrous and liquid at 25°C and atmospheric pressure.

9. The product according to any of the claims 1 to 6 **characterized in that** composition A is an aqueous composition having a pH in the range of 7 to 12, preferably in the range of 7.5 to 11.5, more preferably in the range of 8 to 11.

10. The product according to any of the preceding claims **characterized in that** one or more compound(s) according to group c) is ammonium sulfate, ammonium chloride, ammonium acetate, ammonium benzoate, ammonium carbamate, ammonium chloride, ammonium ferric pentetate, ammonium fluoride, ammonium iodide, ammonium bromide, ammonium lactate, ammonium nitrate, ammonium phosphate, diammonium phosphate, triammonium phosphate, ammonium propionate, ammonium sulfite, ammonium thiocyanate, ammonium xylenesulfonate, ammonium citrate, diammonium citrate, triammonium citrate, and/or their mixtures, preferably one or more compound(s) according to group c) is ammonium sulfate, ammonium chloride, ammonium phosphate, diammonium phosphate, triammonium phosphate, and/or their mixtures, more preferably one or more compound(s) according to group c) is ammonium sulfate.

11. The product according to any of the preceding claims **characterized in that** the total concentration of one or more compound(s) according to group c) in composition B is in the range of 0.1% to 15% by weight, preferably in the range of 0.5% to 10% by weight, more preferably in the range of 0.75% to 8% by weight, still more preferably in the range of 1% to 6% by weight, calculated to the total weight of composition B.

12. The product according to any of the preceding claims **characterized in that** the pH of the ready-to-use composition yielded by mixing compositions A and B is in the range of 7 to 12, preferably in the range of 7.5 to 11.5, more preferably in the range of 8 to 11.

13. The product according to any of the preceding claims **characterized in that** the compositions A and B have a mixing ratio by weight in the range of 0.1 to 10, preferably in the range of 0.2 to 5, more preferably in the range of 0.3 to 3, still more preferably they have a mixing ratio by weight of 1.

14. A method for dyeing of keratin fibers, preferably human keratin fibers, more preferably human hair, comprising the steps of:
i) providing composition A as defined in any of the claims 1 to 13,
ii) providing composition B as defined in any of the claims 1 to 13,
iii) mixing the compositions of steps i) and ii) directly prior to application onto keratin fibers to yield a ready-to-use composition having a pH in the range of 7 to 12,
iv) applying the ready-to-use composition onto keratin fibers for a time period of 1 min to 60 min,
v) optionally rinsing off the keratin fibers.

15. Use of a composition as defined as composition B in any of the claims 1 to 13 for enhancing color intensity and wash fastness of one or more direct dye(s) on keratin fibers, preferably human keratin fibers, more preferably human hair, with the provision that the composition B is mixed with composition A as defined in any of the claims 1 to 13 directly prior to application onto keratin fibers.

## Patentansprüche

1. Färbeprodukt für Keratinfasern, bevorzugt menschliche Keratinfasern, mehr bevorzugt menschliches Haar, umfassend mindestens die Zusammensetzungen A und B, **dadurch gekennzeichnet, dass**
- die Zusammensetzung A a) einen oder mehr Direktfarbstoff(e) für Haar und b) ein oder mehr Alkalisierungsmittel umfasst, und
- die Zusammensetzung B eine wässrige Zusammensetzung mit einem pH-Wert im Bereich von 1 bis 6 ist und c) ein oder mehr Ammoniumsalz(e) der folgenden allgemeinen Formel umfasst:
NH₄⁺ Y⁻,
mit der Maßgabe, dass Y⁻ ein Anion mit Ausnahme von OH⁻und CO₃²⁻ ist,
wobei die Gesamtkonzentration an Oxidationsmitteln in der Zusammensetzung B 1 Gew.-% oder weniger, bevorzugt 0,1 Gew.-% oder weniger, berechnet auf das Gesamtgewicht der Zusammensetzung B, beträgt, wobei die Zusammensetzung B mehr bevorzugt frei von Oxidationsmitteln ist.

2. Produkt gemäß Anspruch 1, **dadurch gekennzeichnet, dass** die Gesamtkonzentration an Oxidationsmitteln in der Zusammensetzung A 1 Gew.-% oder weniger, bevorzugt 0,1 Gew.-% oder weniger, berechnet auf das Gesamtgewicht der Zusammensetzung A, beträgt, wobei die Zusammensetzung A mehr bevorzugter frei von Oxidationsmitteln ist, und das Färbeprodukt noch mehr bevorzugt ein nicht-oxidatives Färbeprodukt ist.

3. Produkt gemäß einem der Ansprüche 1 und/oder 2, **dadurch gekennzeichnet, dass** eine oder mehr Verbindung(en) gemäß Gruppe a) ein oder mehr nichtionische(r) und/oder anionische(r) Direktfarbstoff(e) und/oder deren Salz(e) und/oder deren Mischungen ist/sind, wobei eine oder mehr Verbindung(en) gemäß Gruppe a) bevorzugt Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Acid Red 52, DC Violet 2, DC Red 33, DC Orange 4, DC Red 27, DC Yellow 10, HC Blue 18, HC Red 18, HC Yellow 16, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 und Basic Orange 31 ist/sind, eine oder mehr Verbindung(en) gemäß Gruppe a) bevorzugt eine oder mehr von Basic Red 51, Basic Yellow 87, Basic Orange 31, HC Blue 17 und Basic Blue 124, HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, HC Blue 18, HC Red 18, HC Yellow 16, Disperse Black 9, Acid Yellow 1 und/oder deren Salz(en) und/oder deren Mischungen ist/sind, eine oder mehr Verbindung(en) gemäß Gruppe a) mehr bevorzugt aus HC Blue 18, HC Red 18, HC Yellow 16, Disperse Black 9, Acid Yellow 1 und/oder deren Salz(en) und/oder deren Mischungen ausgewählt ist/sind, eine oder mehr Verbindung(en) gemäß Gruppe a) noch mehr bevorzugt aus HC Blue 18, HC Red 18, HC Yellow 16 und/oder deren Salz(en) und/oder deren Mischungen ausgewählt ist/sind.

4. Produkt gemäß einem der vorstehenden Ansprüche, **dadurch gekennzeichnet, dass** die Gesamtkonzentration der einen oder mehr Verbindung(en) gemäß Gruppe a) in der Zusammensetzung A im Bereich von 0,05 bis 15 Gew.-%, bevorzugt im Bereich von 0,1 bis 12 Gew.-%, mehr bevorzugt im Bereich von 0,25 bis 10 Gew.-%, berechnet auf das Gesamtgewicht der Zusammensetzung A, liegt.

5. Produkt gemäß einem der vorstehenden Ansprüche, **dadurch gekennzeichnet, dass** eine oder mehr Verbindung(en) gemäß Gruppe b) aus einem oder mehr organischen Alkalisierungsmittel(n) und/oder deren Salz(en) und/oder Ammoniumhydroxid ausgewählt ist/sind, wobei eine oder mehr Verbindung(en) gemäß Gruppe b) bevorzugt ein oder mehr Alkanolamin(e) und/oder dessen/deren Salz(e) und/oder Ammoniumhydroxid ist/sind, eine oder mehr Verbindung(en) gemäß Gruppe b) mehr bevorzugt aus Monoethanolamin, Diethanolamin, Monoethanolmethylamin, Monoethanoldimethylamin, Diethanolmethylamin, Monoethanolethylamin, Monoethanoldiethylamin, Diethanolethylamin, Monoethanolpropylamin, Monoethanoldipropylamin, Diethanolpropylamin, Monoethanolbutylamin, Diethanolbutylamin, Trimethylamin, Triethylamin, 2-Amino-2-methylpropanol, Tris-(hydroxymethyl)-aminomethan, Ammoniumhydroxid und/oder deren Mischungen ausgewählt ist/sind, eine oder mehr Verbindung(en) gemäß Gruppe b) am meisten bevorzugt aus Monoethanolamin, 2-Amino-2-methylpropanol, Tris-(hydroxymethyl)-aminomethan, Ammoniumhydroxid und/oder deren Mischungen ausgewählt ist/sind.

6. Produkt gemäß einem der vorstehenden Ansprüche, **dadurch gekennzeichnet, dass** die Gesamtkonzentration der einen oder mehr Verbindung(en) gemäß Gruppe b) in der Zusammensetzung A im Bereich von 0,1 bis 30 Gew.-%, bevorzugt im Bereich von 0,25 bis 25 Gew.-%, mehr bevorzugt im Bereich von 0,5 bis 20 Gew.-%, berechnet auf das Gesamtgewicht der Zusammensetzung A, liegt.

7. Produkt gemäß einem der vorstehenden Ansprüche, **dadurch gekennzeichnet, dass** die Zusammensetzung A eine Pulverzusammensetzung ist und Wasser zu 10 Gew.-% oder weniger, bevorzugt 1 Gew.-% oder weniger, mehr bevorzugt 0,1 Gew.-% oder weniger, berechnet auf das Gesamtgewicht der Zusammensetzung A, enthält, die Zusammensetzung A noch mehr bevorzugt eine wasserfreie Pulverzusammensetzung ist.

8. Produkt gemäß einem der Ansprüche 1 bis 6, **dadurch gekennzeichnet, dass** die Zusammensetzung A bei 25°C und Atmosphärendruck eine flüssige Zusammensetzung ist, die ein oder mehr organische Lösungsmittel und weniger als 10 Gew.-% Wasser, bevorzugt 1 Gew.-% Wasser oder weniger, mehr bevorzugt 0,1 Gew.-% Wasser oder weniger, berechnet auf das Gesamtgewicht der Zusammensetzung A, umfasst, die Zusammensetzung A noch mehr bevorzugt ein oder mehr organische Lösungsmittel umfasst, wasserfrei ist und bei 25°C und Atmosphärendruck flüssig ist.

9. Produkt gemäß einem der Ansprüche 1 bis 6, **dadurch gekennzeichnet, dass** die Zusammensetzung A eine wässrige Zusammensetzung ist, die einen pH-Wert im Bereich von 7 bis 12, bevorzugt im Bereich von 7,5 bis 11,5, mehr bevorzugt im Bereich von 8 bis 11, aufweist.

10. Produkt gemäß einem der vorstehenden Ansprüche, **dadurch gekennzeichnet, dass** eine oder mehr Verbindung(en) gemäß Gruppe c) Ammoniumsulfat, Ammoniumchlorid, Ammoniumacetat, Ammoniumbenzoat, Ammoniumcarbamat, Ammoniumchlorid, Ammoniumeisen(III)-pentetat, Ammoniumfluorid, Ammoniumiodid, Ammoniumbromid, Ammoniumlactat, Ammoniumnitrat, Ammoniumphosphat, Diammoniumphosphat, Triammoniumphosphat, Ammoniumpropionat, Ammoniumsulfit, Ammoniumthiocyanat, Ammoniumxylensulfonat, Ammoniumcitrat, Diammoniumcitrat, Triammoniumcitrat und/oder deren Mischungen ist/sind, wobei eine oder mehr Verbindung(en) gemäß Gruppe c) bevorzugt Ammoniumsulfat, Ammoniumchlorid, Ammoniumphosphat, Diammoniumphosphat, Triammoniumphosphat und/oder deren Mischungen ist/sind, eine oder mehr Verbindung(en) gemäß Gruppe c) mehr bevorzugt Ammoniumsulfat ist/sind.

11. Produkt gemäß einem der vorstehenden Ansprüche, **dadurch gekennzeichnet, dass** die Gesamtkonzentration der einen oder mehr Verbindung(en) gemäß Gruppe c) in der Zusammensetzung B im Bereich von 0,1 bis 15 Gew.-%, bevorzugt im Bereich von 0,5 bis 10 Gew.-%, mehr bevorzugt im Bereich von 0,75 bis 8 Gew.-%, noch mehr bevorzugt im Bereich von 1 bis 6 Gew.-%, berechnet auf das Gesamtgewicht der Zusammensetzung B, liegt.

12. Produkt gemäß einem der vorstehenden Ansprüche, **dadurch gekennzeichnet, dass** der pH-Wert der gebrauchsfertigen Zusammensetzung, erhalten durch Mischen der Zusammensetzungen A und B, im Bereich von 7 bis 12, bevorzugt im Bereich von 7,5 bis 11,5, mehr bevorzugt im Bereich von 8 bis 11, liegt.

13. Produkt gemäß einem der vorstehenden Ansprüche, **dadurch gekennzeichnet, dass** die Zusammensetzungen A und B ein Gewichtsmischungsverhältnis im Bereich von 0,1 bis 10, bevorzugt im Bereich von 0,2 bis 5, mehr bevorzugt im Bereich von 0,3 bis 3, aufweisen, sie noch mehr bevorzugt ein Gewichtsmischungsverhältnis von 1 aufweisen.

14. Verfahren zum Färben von Keratinfasern, bevorzugt menschlichen Keratinfasern, mehr bevorzugt menschlichem Haar, umfassend die Schritte:
i) das Bereitstellen der Zusammensetzung A, wie in einem der Ansprüche 1 bis 13 definiert,
ii) das Bereitstellen der Zusammensetzung B, wie in einem der Ansprüche 1 bis 13 definiert,
iii) das Mischen der Zusammensetzungen aus den Schritten i) und ii) unmittelbar vor dem Auftragen auf Keratinfasern, um eine gebrauchsfertige Zusammensetzung mit einem pH-Wert im Bereich von 7 bis 12 zu erhalten,
iv) das Aufbringen der gebrauchsfertigen Zusammensetzung auf Keratinfasern für einen Zeitraum von 1 min bis 60 min,
v) optional das Ausspülen der Keratinfasern.

15. Verwendung einer Zusammensetzung, wie sie als Zusammensetzung B in einem der Ansprüche 1 bis 13 definiert ist, zur Verbesserung der Farbintensität und der Waschechtheit eines oder mehrerer Direktfarbstoffe(s) auf Keratinfasern, bevorzugt menschlichen Keratinfasern, mehr bevorzugt menschlichem Haar, mit der Maßgabe, dass die Zusammensetzung B unmittelbar vor dem Aufbringen auf Keratinfasern mit der Zusammensetzung A, wie in einem der Ansprüche 1 bis 13 definiert, gemischt wird.

## Revendications

1. Produit de coloration pour fibres de kératine, préférablement des fibres de kératine humaines, plus préférablement des cheveux humains, comprenant au moins les compositions A et B, **caractérisé en ce que**
- la composition A comprend a) un ou plusieurs colorant(s) capillaire(s) direct(s), et b) un ou plusieurs agent(s) alcalinisant(s), et
- la composition B est une composition aqueuse présentant un pH dans la plage de 1 à 6 et comprend c) un ou plusieurs sel(s) d'ammonium de formule générale suivante :
NH₄⁺ Y⁻,
à condition que Y⁻ soit un anion à l'exception de OH- et CO₃²⁻,
dans lequel la concentration totale en agents oxydants dans la composition B est de 1 % en poids ou moins, préférablement de 0,1 % en poids ou moins, calculée par rapport au poids total de la composition B, plus préférablement la composition B est exempte d'agents oxydants.

2. Produit selon la revendication 1 **caractérisé en ce que** la concentration totale en agents oxydants dans la composition A est de 1 % en poids ou moins, préférablement de 0,1 % en poids ou moins, calculée par rapport au poids total de la composition A, encore plus préférablement la composition A est exempte d'agents oxydants, encore plus préférablement le produit de coloration est un produit de coloration non oxydante.

3. Produit selon l'une quelconque des revendications 1 et/ou 2, **caractérisé en ce qu'**un ou plusieurs composé(s) du groupe a) est(sont) un ou plusieurs colorant(s) direct(s) non ionique(s) et/ou anionique(s), et/ou leurs sels, et/ou leurs mélanges, préférablement un ou plusieurs composé(s) du groupe a) est(sont) Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Acid Red 52, DC Violet 2, DC Red 33, DC Orange 4, DC Red 27, DC Yellow 10, HC Blue 18, HC Red 18, HC Yellow 16, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12, Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 et Basic Orange 31, préférablement un ou plusieurs composé(s) du groupe a) est(sont) un ou plusieurs parmi Basic Red 51, Basic Yellow 87, Basic Orange 31, HC Blue 17 et Basic Blue 124, HC Blue No.2, HC Blue No.4, HC Blue No.5, HC Blue No.6, HC Blue No.7, HC Blue No.8, HC Blue No.9, HC Blue No.10, HC Blue No. 11, HC Blue No. 12, HC Blue No. 13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No. 10, HC Red No. 11, HC Red No. 13, HC Red No.54, HC Red No. 14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No. 10, HC Yellow No. 11, HC Yellow No.12, HC Yellow No. 13, HC Yellow No. 14, HC Yellow No. 15, HC Blue 18, HC Red 18, HC Yellow 16, Disperse Black 9, Acid Yellow 1, et/ou leurs sels, et/ou leurs mélanges, plus préférablement un ou plusieurs composé(s) du groupe a) est(sont) sélectionné(s) parmi HC Blue 18, HC Red 18, HC Yellow 16, Disperse Black 9, Acid Yellow 1, et/ou leurs sels, et/ou leurs mélanges, encore plus préférablement un ou plusieurs composé(s) du groupe a) est(sont) sélectionné(s) parmi HC Blue 18, HC Red 18, HC Yellow 16, et/ou leurs sels, et/ou leurs mélanges.

4. Produit selon l'une quelconque des revendications précédentes, **caractérisé en ce que** la concentration totale d'un ou plusieurs composé(s) du groupe a) dans la composition A est dans la plage de 0,05 % à 15 % en poids, préférablement dans la plage de 0,1 % à 12 % en poids, plus préférablement dans la plage de 0,25 % à 10 % en poids, calculée par rapport au poids total de la composition A.

5. Produit selon l'une quelconque des revendications précédentes, **caractérisé en ce qu'**un ou plusieurs composé(s) du groupe b) est(sont) sélectionné(s) parmi un ou plusieurs agent(s) alcalinisant(s) organique(s), et/ou leurs sels, et/ou l'hydroxyde d'ammonium, préférablement un ou plusieurs composé(s) du groupe b) est(sont) une ou plusieurs alcanolamine(s) et/ou son/leurs sel(s) et/ou l'hydroxyde d'ammonium, plus préférablement un ou plusieurs composé(s) du groupe b) est(sont) sélectionné(s) parmi monoéthanolamine, diéthanolamine, monoéthanol méthylamine, monoéthanol diméthylamine, diéthanolméthylamine, monoéthanoléthylamine, monoéthanoldiéthylamine, diéthanoléthylamine, monoéthanolpropylamine, monoéthanoldipropylamine, diéthanolpropylamine, monoéthanolbutylamine, diéthanolbutylamine, triméthylamine, triéthylamine, 2-amino-2-méthylpropanol, tris-(hydroxyméthyl)-aminométhane, hydroxyde d'ammonium, et/ou leurs mélanges, le plus préférablement un ou plusieurs composé(s) du groupe b) est(sont) sélectionnés parmi monoéthanolamine, 2-amino-2-méthylpropanol, tris-(hydroxyméthyl)-aminométhane, hydroxyde d'ammonium, et/ou leurs mélanges.

6. Produit selon l'une quelconque des revendications précédentes, **caractérisé en ce que** la concentration totale d'un ou plusieurs composé(s) du groupe b) dans la composition A est dans la plage de 0,1 % à 30 % en poids, préférablement dans la plage de 0,25 % à 25 % en poids, plus préférablement dans la plage de 0,5 % à 20 % en poids, calculée par rapport au poids total de la composition A.

7. Produit selon l'une quelconque des revendications précédentes, **caractérisé en ce que** la composition A est une composition pulvérulente et comprend de l'eau à 10 % en poids ou moins, préférablement à 1 % en poids ou moins, plus préférablement à 0,1 % en poids ou moins, calculé par rapport au poids total de la composition A, encore plus préférablement la composition A est une composition pulvérulente anhydre.

8. Produit selon l'une quelconque des revendications 1 à 6, **caractérisé en ce que** la composition A est une composition liquide à 25 °C et à pression atmosphérique comprenant un ou plusieurs solvant(s) organique(s) et moins de 10 % en poids d'eau, préférablement 1 % en poids d'eau ou moins, plus préférablement 0,1 % en poids d'eau ou moins, calculé par rapport au poids total de la composition A, encore plus préférablement la composition A comprenant un ou plusieurs solvant(s) organique(s) est anhydre et liquide à 25 °C et à pression atmosphérique.

9. Produit selon l'une quelconque des revendications 1 à 6, **caractérisé en ce que** la composition A est une composition aqueuse présentant un pH dans la plage de 7 à 12, préférablement dans la plage de 7,5 à 11,5, plus préférablement dans la plage de 8 à 11.

10. Produit selon l'une quelconque des revendications précédentes, **caractérisé en ce qu'**un ou plusieurs composé(s) du groupe c) est(sont) sulfate d'ammonium, chlorure d'ammonium, acétate d'ammonium, benzoate d'ammonium, carbamate d'ammonium, chlorure d'ammonium, pentétate ferrique d'ammonium, fluorure d'ammonium, iodure d'ammonium, bromure d'ammonium, lactate d'ammonium, nitrate d'ammonium, phosphate d'ammonium, phosphate de diammonium, phosphate de triammonium, propionate d'ammonium, sulfite d'ammonium, thiocyanate d'ammonium, xylènesulfonate d'ammonium, citrate d'ammonium, citrate de diammonium, citrate de triammonium, et/ou leurs mélanges, préférablement un ou plusieurs composé(s) du groupe c) est(sont) sulfate d'ammonium, chlorure d'ammonium, phosphate d'ammonium, phosphate de diammonium, phosphate de triammonium, et/ou leurs mélanges, plus préférablement un ou plusieurs composé(s) du groupe c) est(sont) le sulfate d'ammonium.

11. Produit selon l'une quelconque des revendications précédentes, **caractérisé en ce que** la concentration totale d'un ou plusieurs composé(s) du groupe c) dans la composition B est dans la plage de 0,1 % à 15 % en poids, préférablement dans la plage de 0,5 % à 10 % en poids, plus préférablement dans la plage de 0,75 % à 8 % en poids, encore plus préférablement dans la plage de 1 % à 6 % en poids, calculée par rapport au poids total de la composition B.

12. Produit selon l'une quelconque des revendications précédentes, **caractérisé en ce que** le pH de la composition prête à l'emploi obtenue en mélangeant les compositions A et B est dans la plage de 7 à 12, préférablement dans la plage de 7,5 à 11,5, plus préférablement dans la plage de 8 à 11.

13. Produit selon l'une quelconque des revendications précédentes, **caractérisé en ce que** les compositions A et B présentent un rapport de mélange en poids dans la plage de 0,1 à 10, préférablement dans la plage de 0,2 à 5, plus préférablement dans la plage de 0,3 à 3, encore plus préférablement elles présentent un rapport de mélange en poids de 1.

14. Procédé de coloration de fibres de kératine, préférablement de fibres de kératine humaines, plus préférablement de cheveux humains, comprenant les étapes suivantes :
i) la fourniture d'une composition A telle que définie dans l'une quelconque des revendications 1 à 13,
ii) la fourniture d'une composition B telle que définie dans l'une quelconque des revendications 1 à 13,
iii) le mélange des compositions des étapes i) et ii) directement avant application sur les fibres de kératine afin d'obtenir une composition prête à l'emploi présentant un pH dans la plage de 7 à 12,
iv) l'application de la composition prête à l'emploi sur des fibres de kératine pendant une période de temps de 1 min à 60 min,
v) facultativement, le rinçage des fibres de kératine.

15. Utilisation d'une composition telle que définie en tant que composition B dans l'une quelconque des revendications 1 à 13 pour augmenter l'intensité de la couleur et la tenue au lavage d'un ou plusieurs colorant(s) direct(s) sur des fibres de kératine, préférablement des fibres de kératine humaines, plus préférablement des cheveux humains, à condition que la composition B soit mélangée avec la composition A telle que définie dans l'une quelconque des revendications 1 à 13 directement avant application sur des fibres de kératine.
